# NOUVEAU FASCICULE DE BREVET EUROPEEN

(11) **EP 2 072 034 B2**
(45) Date de publication et mention de la décision concernant l'opposition: **06.12.2017**
(45) Mention de la délivrance du brevet: 12.03.2014
(21) Numéro de dépôt: 08172444.5
(22) Date de dépôt: 19.12.2008
(51) Int. Cl.: A61K 8/41, A61Q 5/08

(54) **Procédé de coloration directe éclaircissante ou d'oxydation en présence d'une amine organique particulière et dispositif**
Verfahren zum direkten aufhellenden oder oxidierenden Färben in Gegenwart eines organischen Amins und Vorrichtung hierfür
Process for lightening direct dyeing or oxidation dyeing in the presence of a particular organic amine, device therefore and anhydrous composition

(30) Priorité: 21.12.2007 FR 0760273
(43) Date de publication de la demande: 24.06.2009
(73) Titulaire: L'Oréal, 75008 Paris (FR)
(72) Inventeur: Hercouet, Leïla, 93360 Neuilly Plaisance (FR); Giafferi, Marie, 93250 Villemonble (FR); Lagrange, Alain, 77700 Coupvray (FR)
(74) Mandataire: Wattremez, Catherine

(56) Documents cités:
- EP-A- 1 291 006
- EP-A- 1 438 951
- EP-A- 1 598 052

## Description

La présente invention a pour objet un procédé de coloration de fibres kératiniques humaines en présence d'un agent oxydant, comprenant la mise en oeuvre d'une composition anhydre (A) cosmétique comprenant un ou plusieurs corps gras, le ou les corps gras étant choisis parmi l'huile de vaseline, les polydécènes ou leurs mélanges, la teneur en corps en corps gras étant comprise entre 10 et 99 % en poids, par rapport au poids de la composition (A), et un ou plusieurs tensioactifs, une composition oxydante (B), une composition (C) comprenant un ou plusieurs colorants et une ou plusieurs amines organiques dont le pKb à 25°C est inférieur à 12, caractérisé en ce que l'on applique une composition obtenue par mélange extemporané avant application, des compositions (A), (B) et (C).

Parmi les méthodes de coloration des fibres kératiniques humaines, telles que les cheveux, on peut citer la coloration d'oxydation ou permanente. Plus particulièrement, ce mode de coloration met en oeuvre un ou plusieurs précurseurs de colorant d'oxydation, habituellement une ou plusieurs bases d'oxydation éventuellement associées à un ou plusieurs coupleurs.

En général, des bases d'oxydation sont choisies parmi les ortho- ou paraphénylènediamines, les ortho- ou para-aminophénols ainsi que des composés hétérocycliques. Ces bases d'oxydation sont des composés incolores ou faiblement colorés qui, associés à des produits oxydants, permettent d'accéder à des espèces colorées, par un processus de condensation oxydative.

Bien souvent, on fait varier les nuances obtenues avec ces bases d'oxydation en les associant à un ou plusieurs coupleurs, ces derniers étant choisis notamment parmi les méta-diamines aromatiques, les méta-aminophénols, les méta-diphénols et certains composés hétérocycliques, tels que des composés indoliques.

La variété des molécules mises en jeu au niveau des bases d'oxydation et des coupleurs permet l'obtention d'une riche palette de couleurs.

On connaît également la coloration directe ou semi-permanente. Le procédé classiquement utilisé en coloration directe consiste à appliquer sur les fibres kératiniques des colorants directs qui sont des molécules colorées et colorantes, ayant une affinité pour les fibres, à laisser pauser pour permettre aux molécules de pénétrer, par diffusion, à l'intérieur de la fibre, puis à les rincer.

Les colorants directs généralement employés sont choisis parmi les colorants directs nitrés benzéniques, anthraquinoniques, nitropyridiniques, azoïques, méthiniques, azométhiniques, xanthéniques, acridiniques, aziniques ou triarylméthaniques.

Ce type de procédé ne nécessite pas l'emploi d'un agent oxydant pour développer la coloration. Cependant, il n'est pas exclu d'en mettre en oeuvre afin d'obtenir avec la coloration, un effet d'éclaircissement. On parle alors de coloration directe ou semi-permanente en conditions éclaircissantes.

EP-A-1 438 951 décrit des compositions cosmétiques anhydres contenant un corps gras (myristate d'isopropyle) et un tensioactif. Une composition oxydante est mélangée avec la composition anhydre pour former la composition de décoloration.

EP-A-1 598 052 divulgue des compositions d'éclaircissement et de coloration de fibres kératiniques contenant une composition anhydre qui contient un corps gras, une composition contenant des corps gras, des tensioactifs et des colorants et une composition oxydante.

EP-A-1 291 006 décrit des compositions aqueuses cosmétiques pour l'éclaircissement et la coloration de fibres kératiniques contenant des corps gras, des tensioactifs et des amines organiques.

Les procédés de coloration permanente ou encore semi-permanente en conditions éclaircissantes, consistent donc à employer avec la composition tinctoriale, une composition aqueuse comprenant au moins un agent oxydant, en condition de pH alcalin dans la grande majorité des cas. Cet agent oxydant a pour rôle de dégrader la mélanine des cheveux, ce qui, en fonction de la nature de l'agent oxydant présent, conduit à un éclaircissement plus ou moins prononcé des fibres. Ainsi, pour un éclaircissement relativement faible, l'agent oxydant est généralement le peroxyde d'hydrogène. Lorsqu'un éclaircissement plus important est recherché, on met habituellement en oeuvre des sels peroxygénés, comme des persulfates par exemple, en présence de peroxyde d'hydrogène.

L'une des difficultés vient du fait que ces procédés sont mis en oeuvre dans des conditions alcalines et que l'agent alcalin le plus couramment utilisé est l'ammoniaque. L'ammoniaque est particulièrement avantageux dans ce type de procédés. En effet, il permet d'ajuster le pH de la composition à un pH alcalin pour permettre l'activation de l'agent oxydant. Mais cet agent provoque également un gonflement de la fibre kératinique, avec une ouverture des écailles, ce qui favorise la pénétration de l'oxydant, ainsi que des colorants, essentiellement les colorants d'oxydation, à l'intérieur de la fibre, et donc augmente l'efficacité de la réaction de coloration.

Or cet agent alcalinisant est très volatil, ce qui occasionne des désagréments à l'utilisateur du fait de l'odeur caractéristique forte, plutôt incommodante de l'ammoniac qui se dégage durant le procédé.

De plus, la quantité d'ammoniac dégagée nécessite l'emploi de teneurs plus importantes que nécessaires pour compenser cette perte. Cela n'est pas sans conséquence pour l'utilisateur qui reste non seulement incommodé par l'odeur mais qui peut également être confronté à des risques plus importants d'intolérance, comme par exemple une irritation du cuir chevelu (picotements).

Quant à l'option de purement et simplement remplacer en totalité ou en partie l'ammoniaque par un ou plusieurs autres agents alcalinisants classiques, celle-ci ne conduit pas à des compositions aussi efficaces que celles à base d'ammoniaque, notamment parce que ces agents alcalinisants ne conduisent pas un éclaircissement suffisant des fibres pigmentées en présence de l'agent oxydant.

L'un des objectifs de la présente invention est de proposer des procédés de coloration mis en oeuvre en présence d'un agent oxydant qui n'ont pas les inconvénients des procédés existants, dus à la présence de teneurs importantes en ammoniaque, tout en restant au moins aussi efficaces, tant sur le plan de la puissance de la coloration obtenue, que de la chromaticité, de l'homogénéité de la coloration le long de la fibre. En particulier, le procédé selon l'invention conduit à des colorations puissantes permettant la couverture de 100 % des cheveux blancs.

Ces buts et d'autres sont atteints par la présente invention qui a donc pour objet un procédé de coloration de fibres kératiniques humaines en présence d'un agent oxydant dans lequel on applique sur lesdites fibres :
1. (a) une composition anhydre (A) cosmétique comprenant un ou plusieurs corps gras, le ou les corps gras étant choisis parmi l'huile de vaseline, les polydécènes ou leurs mélanges, la teneur en corps en corps gras étant comprise entre 10 et 99 % en poids, par rapport au poids de la composition (A),, et un ou plusieurs tensioactifs ;
2. (b) une composition (B) comprenant un ou plusieurs agents oxydants ;
3. (c) une composition (C) comprenant un ou plusieurs colorants d'oxydation, un
ou plusieurs colorants directs, ou leurs mélanges et une ou plusieurs amines organiques dont le pKb est inférieur à 12 à 25°C,
caractérisé en ce que l'on applique une composition obtenue par mélange extemporané avant application, des compositions (A), (B) et (C).

D'autres caractéristiques et avantages de l'invention apparaîtront plus clairement à la lecture de la description et des exemples qui suivent.

Dans ce qui va suivre, et à moins d'une autre indication, les bornes d'un domaine de valeurs sont comprises dans ce domaine.

Les fibres kératiniques humaines traitées par le procédé selon l'invention sont de préférence les cheveux.

Comme indiqué auparavant, le procédé de coloration est mis en oeuvre en présence d'une composition anhydre (A).

Plus particulièrement, on entend par composition anhydre, au sens de l'invention, une composition présentant une teneur en eau inférieure à 5 % en poids, de préférence inférieure à 2% en poids et de manière encore plus préférée inférieure à 1 % en poids par rapport au poids de ladite composition. Il est à noter que l'eau peut aussi se trouver sous forme d'eau liée, comme l'eau de cristallisation des sels ou des traces d'eau absorbée par les matières premières utilisées dans la réalisation des compositions selon l'invention.

Ainsi que cela a été mentionné, la composition cosmétique anhydre (A) comprend un ou plusieurs corps gras.

Par corps gras, on entend, un composé organique insoluble dans l'eau à température ordinaire (25°C) et à pression atmosphérique (760 mm de Hg) (solubilité inférieure à 5% et de préférence à 1% encore plus préférentiellement à 0.1 %). En outre, les corps gras sont solubles dans les solvants organiques dans les mêmes conditions de température et de pression, comme par exemple le chloroforme, l'éthanol ou le benzène.

De préférence, le corps gras est un composé liquide à la température de 25°C et à la pression atmosphérique.

Le corps gras est choisi parmi l'huile de vaseline, les polydécènes, ou leurs mélanges.

La composition cosmétique anhydre présente une teneur en corps gras comprise entre 10 et 99 % en poids, de préférence entre 20 et 90 % en poids, et encore plus particulièrement entre 25 et 80% en poids, par rapport au poids de la composition anhydre.

La composition anhydre cosmétique (A) comprend également un ou plusieurs tensioactifs.

Plus particulièrement, le ou les tensioactifs sont choisis parmi les tensioactifs non ioniques ou parmi les tensioactifs anioniques.

Les tensioactifs anioniques sont plus spécialement choisis parmi les sels (en particulier sels de métaux alcalins, notamment de sodium, sels d'ammonium, sels d'amines, sels d'aminoalcools ou sels de métaux alcalino-terreux comme le magnésium) des composés suivants :
- les alkylsulfates, les alkyléthersulfates, alkylamidoéthersulfates, alkylarylpolyéthersulfates, monoglycérides sulfates ;
- les alkylsulfonates, alkylamidesulfonates, alkylarylsulfonates, α-oléfine-sulfonates, paraffine-sulfonates ;
- les alkylphosphates, les alkylétherphosphates;
- les alkylsulfosuccinates, les alkyléthersulfosuccinates, les alkylamidesulfosuccinates; les alkylsulfosuccinamates ;
- les alkylsulfoacétates ;
- les acylsarcosinates ; les acyliséthionates et les N-acyltaurates ;
- les sels d'acides gras tels que les acides oléique, ricinoléique, palmitique, stéarique, les acides d'huile de coprah ou d'huile de coprah hydrogénée ;
- les sels d'acides d'alkyl D galactoside uroniques ;
- les acyl-lactylates ;
- les sels des acides alkyléther carboxyliques polyoxyalkylénés, des acides alkylaryléther carboxyliques polyoxyalkylénés, des acides alkylamidoéther carboxyliques polyoxyalkylénés, en particulier ceux comportant de 2 à 50 groupements oxyde d'éthylène ;
- et leurs mélanges.

Il est à noter que le radical alkyle ou acyle de ces différents composés comporte avantageusement de 6 à 24 atomes de carbone, et de préférence de 8 à 24 atomes de carbone, et le radical aryle désignant de préférence un groupement phényle ou benzyle.

Les tensioactifs non ioniques sont plus particulièrement choisis parmi les tensioactifs non ioniques mono ou poly- oxyalkylénés, mono- ou poly- glycérolés. Les motifs oxyalkylénés sont plus particulièrement des motifs oxyéthylénés, oxypropylénés, ou leur combinaison, de préférence oxyéthylénés.

A titre d'exemples de tensioactifs non ioniques oxyalkylénés, on peut citer :
- les alkyl(C₈-C₂₄)phénols oxyalkylénés,
- les alcools en C₈-C₃₀, saturés ou non, linéaires ou ramifiés, oxyalkylénés,
- les amides, en C₈-C₃₀, saturés ou non, linéaires ou ramifiés, oxyalkylénés,
- les amines en C₈-C₃₀, saturées ou non, linéaires ou ramifiées, oxyalkylénées,
- les esters d'acides en C₈-C₃₀, saturés ou non, linéaires ou ramifiés, et de polyéthylèneglycols,
- les esters d'acides en C₈-C₃₀, saturés ou non, linéaires ou ramifiés, et de sorbitol polyoxyéthylénés,
- les huiles végétales oxyéthylénées, saturées ou non,
- les condensats d'oxyde d'éthylène et/ou d'oxyde de propylène, entre autres, seuls
ou en mélanges.

Les tensioactifs présentant un nombre de moles d'oxyde d'éthylène et/ou de propylène compris entre 1 et 50, de préférence entre 2 et 30. De manière avantageuse, les tensioactifs non ioniques ne comprennent pas de motifs oxypropylénés.

Conformément à un mode de réalisation préféré de l'invention, les tensioactifs non ioniques oxyalkylénés sont choisis parmi les alcools en C₈-C₃₀, oxyéthylénés, les amines en C₈-C₃₀, oxyéthylénées.

A titre d'exemple de tensioactifs non ioniques mono- ou poly- glycérolés, on utilise de préférence les alcools en C₈-C₄₀, mono- ou poly- glycérolés.

En particulier, les alcools en C₈-C₄₀ mono- ou poly- glycérolés correspondent à la formule suivante:

RO-[CH₂-CH(CH₂OH)-O]ₘ-H

dans laquelle R représente un radical alkyle ou alcényle, linéaire ou ramifié, en C₈-C₄₀, de préférence en C₈-C₃₀, et m représente un nombre allant de 1 à 30 et de préférence de 1 à 10.

A titre d'exemple de composés convenables dans le cadre de l'invention, on peut citer, l'alcool laurique à 4 moles de glycérol (nom INCI : POLYGLYCERYL-4 LAURYL ETHER), l'alcool laurique à 1,5 moles de glycérol, l'alcool oléique à 4 moles de glycérol (nom INCI : POLYGLYCERYL-4 OLEYL ETHER), l'alcool oléique à 2 moles de glycérol (Nom INCI : POLYGLYCERYL-2 OLEYL ETHER), l'alcool cétéarylique à 2 moles de glycérol, l'alcool cétéarylique à 6 moles de glycérol, l'alcool oléocétylique à 6 moles de glycérol, et l'octadécanol à 6 moles de glycérol.

L'alcool peut représenter un mélange d'alcools au même titre que la valeur de m représente une valeur statistique, ce qui signifie que dans un produit commercial peuvent coexister plusieurs espèces d'alcools gras polyglycérolés sous forme d'un mélange.

Parmi les alcools mono- ou poly-glycérolés, on préfère plus particulièrement utiliser l'alcool en C₈/C₁₀ à une mole de glycérol, l'alcool en C₁₀/C₁₂ à 1 mole de glycérol et l'alcool en C₁₂ à 1,5 mole de glycérol.

De préférence, le tensioactif présent dans la composition anhydre est un tensioactif non ionique.

La teneur en tensioactifs dans la composition anhydre (A), représente plus particulièrement de 0,1 à 50 % en poids, de préférence de 0,5 à 30 % en poids par rapport au poids de la composition anhydre.

La composition anhydre (A) peut également renfermer divers adjuvants utilisés classiquement dans les compositions pour la coloration des cheveux, tels que des polymères anioniques, cationiques, non ioniques, amphotères, zwitterioniques ou leurs mélanges ; des agents épaississants minéraux, et en particulier des charges telles que des argiles, le talc ; des agents épaississants organiques, avec en particulier les épaississants associatifs polymères anioniques, cationiques, non ioniques et amphotères ; des agents antioxydants ; des agents de pénétration ; des agents séquestrants ; des parfums ; des agents dispersants ; des agents filmogènes ; des céramides ; des agents conservateurs ; des agents opacifiants.

Les adjuvants ci dessus sont en général présents en quantité comprise pour chacun d'eux entre 0,01 et 20 % en poids par rapport au poids de la composition (A).

Selon une variante avantageuse de l'invention, la composition anhydre (A) comprend au moins une silice, de préférence à caractère hydrophobe, comme les silices pyrogénées.

Lorsqu'elles sont présentes, les silices représentent de 1 à 30 % en poids par rapport au poids de la composition anhydre (A).

Avantageusement, la composition se présente sous la forme d'un gel ou d'une crème.

Comme indiqué auparavant, le procédé selon l'invention est mis en oeuvre en présence d'une composition (B) comprenant un ou plusieurs agents oxydants.

Plus particulièrement, le ou les agents oxydants sont choisis parmi le peroxyde d'hydrogène, le peroxyde d'urée, les bromates ou ferricyanures de métaux alcalins, les sels peroxygénés comme par exemple les persulfates, les perborates et les percarbonates de métaux alcalins ou alcalino-terreux, et les peracides et leurs précurseurs.

Cet agent oxydant est avantageusement constitué par du peroxyde d'hydrogène et notamment en solution aqueuse (eau oxygénée) dont le titre peut varier, plus particulièrement, de 1 à 40 volumes, et encore plus préférentiellement de 5 à 40 volumes.

En fonction du degré d'éclaircissement souhaité, l'agent oxydant peut également comprendre un agent oxydant choisi de préférence parmi les sels peroxygénés.

De préférence, l'agent oxydant n'est pas choisi parmi les sels peroxygénés et les peracides et précurseurs.

La composition oxydante peut être aqueuse ou non. Par composition aqueuse, on entend une composition comprenant plus de 5 % en poids d'eau, de préférence plus de 10 % en poids d'eau, et de manière encore plus avantageuse plus de 20 % en poids d'eau.

De préférence, la composition (C) est une composition aqueuse.

Elle peut également comprendre un ou plusieurs solvants organiques.

A titre de solvant organique, on peut par exemple citer, les alcanols, linéaires ou ramifiés, en C₂-C₄, tels que l'éthanol et l'isopropanol ; le glycérol ; les polyols et éthers de polyols comme le 2-butoxyéthanol, le propylèneglycol, le dipropylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Le ou les solvants, s'ils sont présents, représentent une teneur allant habituellement de 1 à 40 % en poids par rapport au poids de la composition oxydante (C), et de préférence de 5 à 30 % en poids.

La composition oxydante (B) peut comprendre un ou plusieurs agents acidifiants.

Parmi les agents acidifiants, on peut citer à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Habituellement, le pH de la composition oxydante (B), lorsqu'elle est aqueuse, est inférieur à 7.

La composition oxydante (B) peut également renfermer d'autres ingrédients classiquement employés dans le domaine, comme notamment ceux détaillés auparavant dans le cadre de la composition anhydre (A).

Enfin, la composition oxydante (B) se présente sous diverses formes, comme par exemple une solution, une émulsion ou un gel.

Le procédé selon l'invention est mis en oeuvre en présence d'une composition (C) comprenant un ou plusieurs colorants d'oxydation, un ou plusieurs colorants directs, ou leurs mélanges.

Les colorants d'oxydation sont en général choisis parmi les bases d'oxydation éventuellement combinée(s) à un ou plusieurs coupleurs.

A titre d'exemple, les bases d'oxydation sont choisies parmi les paraphénylènediamines, les bis-phénylalkylènediamines, les para-aminophénols, les ortho-aminophénols, les bases hétérocycliques et leurs sels d'addition.

Parmi les paraphénylènediamines, on peut citer à titre d'exemple, la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-(β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ,-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylène-diamine, la 4-aminophénylpyrrolidine, la 2-thiényl paraphénylènediamine, le 2-β hydroxyéthylamino 5-amino toluène, la 3-hydroxy 1-(4'-aminophényl)pyrrolidine et leurs sels d'addition avec un acide.

Parmi les paraphénylènediamines citées ci-dessus, la paraphénylènediamine, la paratoluylènediamine, la 2-isopropyl paraphénylènediamine, la 2-β-hydroxyéthyl paraphénylènediamine, la 2-β-hydroxyéthyloxy paraphénylène-diamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide sont particulièrement préférées.

Parmi les bis-phénylalkylènediamines, on peut citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diamino phénoxy)-3,6-dioxaoctane, et leurs sels d'addition.

Parmi les para-aminophénols, on peut citer à titre d'exemple, le para-aminophénol, le 4-amino 3-méthyl phénol, le 4-amino 3-fluoro phénol, le 4-amino-3-chlorophénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les ortho-aminophénols, on peut citer à titre d'exemple, le 2-amino phénol, le 2-amino 5-méthyl phénol, le 2-amino 6-méthyl phénol, le 5-acétamido 2-amino phénol, et leurs sels d'addition.

Parmi les bases hétérocycliques, on peut citer à titre d'exemple, les dérivés pyridiniques, les dérivés pyrimidiniques et les dérivés pyrazoliques.

Parmi les dérivés pyridiniques, on peut citer les composés décrits par exemple dans les brevets GB 1 026 978 et GB 1 153 196, comme la 2,5-diamino pyridine, la 2-(4-méthoxyphényl)amino 3-amino pyridine, la 3,4-diamino pyridine, et leurs sels d'addition.

D'autres bases d'oxydation pyridiniques utiles dans la présente invention sont les bases d'oxydation 3-amino pyrazolo-[1,5-a]-pyridines ou leurs sels d'addition décrits par exemple dans la demande de brevet FR 2801308. A titre d'exemple, on peut citer la pyrazolo[1,5-a]pyridin-3-ylamine ; la 2-acétylamino pyrazolo-[1,5-a] pyridin-3-ylamine ; la 2-morpholin-4-yl-pyrazolo[1,5-a]pyridin-3-ylamine ; l'acide 3-amino-pyrazolo[1,5-a]pyridin-2-carboxylique ; la 2-méthoxy-pyrazolo[1,5-a]pyridine-3-ylamino ; le (3-amino-pyrazolo[1,5-a]pyridine-7-yl)-méthanol ; le 2-(3-amino-pyrazolo[1,5-a]pyridine-5-yl)-éthanol ; le 2-(3-amino-pyrazolo[1,5-a]pyridine-7-yl)-éthanol ; le (3-amino-pyrazolo[1,5-a]pyridine-2-yl)-méthanol ; la 3,6-diamino-pyrazolo[1,5-a]pyridine ; la 3,4-diamino-pyrazolo[1,5-a]pyridine ; la pyrazolo[1,5-a]pyridine-3,7-diamine ; la 7-morpholin-4-yl-pyrazolo[1,5-a]pyridin-3-ylamine ; la pyrazolo[1,5-a]pyridine-3,5-diamine ; la 5-morpholin-4-yl-pyrazolo[1,5-a]pyridin-3-ylamine ; le 2-[(3-amino-pyrazolo[1,5-a]pyridin-5-yl)-(2-hydroxyéthyl)-amino]-éthanol ; le 2-[(3-amino-pyrazolo[1,5-a]pyridin-7-yl)-(2-hydroxyéthyl)-amino]-éthanol ; la 3-amino-pyrazolo[1,5-a]pyridine-5-ol ; 3-amino-pyrazolo[1,5-a]pyridine-4-ol ; la 3-amino-pyrazolo[1,5-a]pyridine-6-ol ; la 3-amino-pyrazolo[1,5-a]pyridine-7-ol ; ainsi que leurs sels d'addition.

Parmi les dérivés pyrimidiniques, on peut citer les composés décrits par exemple dans les brevets DE 2359399 ; JP 88-169571 ; JP 05-63124 ; EP 0770375 ou demande de brevet WO 96/15765 comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine et leurs sels d'addition et leurs formes tautomères, lorsqu'il existe un équilibre tautomérique.

Parmi les dérivés pyrazoliques, on peut citer les composés décrits dans les brevets DE 3843892, DE 4133957 et demandes de brevet WO 94/08969, WO 94/08970, FR-A-2 733 749 et DE 195 43 988 comme le 4,5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition. On peut aussi utiliser le 4-5-diamino 1-(β-méthoxyéthyl)pyrazole.

A titre de base hétérocyclique,on peut aussi citer la 2,3 diamino 6,7 dihydro 1 H5H [pyrazolo1,2,a] pyrazol-1-one ou l'un de ses sels.

La composition selon l'invention peut éventuellement comprendre un ou plusieurs coupleurs choisis avantageusement parmi ceux conventionnellement utilisés pour la teinture des fibres kératiniques.

Parmi ces coupleurs, on peut notamment citer les méta-phénylènediamines, les méta-aminophénols, les méta-diphénols, les coupleurs naphtaléniques, les coupleurs hétérocycliques ainsi que leurs sels d'addition.

A titre d'exemple, on peut citer le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthyiamino-3,4-méthylènedioxybenzène, l'a-naphtol, le 2 méthyl-1-naphtol, le 6-hydroxy indole, le 4-hydroxy indole, le 4-hydroxy N-méthyl indole, la 2-amino-3-hydroxy pyridine, la 6- hydroxy benzomorpholine la 3,5-diamino-2,6-diméthoxypyridine, le 1-N-(β-hydroxyéthyl)amino-3,4-méthylène dioxybenzène, le 2,6-bis-(β-hydroxyéthylamino)toluène, la 6-hydroxy indoline, la 2,6-dihydroxy 4-méthyl pyridine, la 1-H 3-méthyl pyrazole 5-one, la 1-phényl 3-méthyl pyrazole 5-one, le 2,6-diméthyl pyrazolo [1,5-b]-1,2,4-triazole, le 2,6-diméthyl [3,2-c]-1,2,4-triazole, le 6-méthyl pyrazolo [1,5-a]-benzimidazole, leurs sels d'addition avec un acide, et leurs mélanges.

D'une manière générale, les sels d'addition des bases d'oxydation et des coupleurs utilisables dans le cadre de l'invention sont notamment choisis parmi les sels d'addition avec un acide tels que les chlorhydrates, les bromhydrates, les sulfates, les citrates, les succinates, les tartrates, les lactates, les tosylates, les benzènesulfonates, les phosphates et les acétates.

La ou les bases d'oxydation représentent chacune avantageusement de 0,0001 à 10 % en poids par rapport au poids total de la composition, et de préférence de 0,005 à 5 % en poids par rapport au poids total de la composition.

La teneur en coupleur(s), s'il(s) est(sont) présent(s), représentent chacun avantageusement de 0,0001 à 10 % en poids par rapport au poids total de la composition, et de préférence de 0,005 à 5 % en poids par rapport au poids total de la composition.

En ce qui concerne les colorants directs, ces derniers sont plus particulièrement choisis parmi les espèces ioniques ou non ioniques, de préférence cationiques ou non ioniques.

A titre d'exemples de colorants directs convenables, on peut citer les colorants directs azoïques ; méthiniques ; carbonyles ; aziniques ; nitrés (hétéro)aryle ; tri-(hétéro)aryle méthanes ; les porphyrines ; les phtalocyanines et les colorants directs naturels, seuls ou en mélanges.

Plus particulièrement, les colorants azoïques comprennent une fonction -N=N- dont les deux atomes d'azote ne sont pas simultanément engagés dans un cycle. Il n'est toutefois pas exclu que l'un des deux atomes d'azote de l'enchaînement -N=N- soit engagé dans un cycle.

Les colorants de la famille des méthines sont plus particulièrement des composés comprenant au moins un enchaînement choisi parmi >C=C< et -N=C< dont les deux atomes ne sont pas simultanément engagés dans un cycle. Il est toutefois précisé que l'un des atomes d'azote ou de carbone des enchaînements peut être engagé dans un cycle. Plus particulièrement, les colorants de cette famille sont issus de composés de type méthine, azométhine, mono- et di-arylméthane, indoamines (ou diphénylamines), indophénols, indoanilines, carbocyanines, azacabocyanines et leurs isomères, diazacarbocyanines et leurs isomères, tétraazacarbocyanines, hémicyanines

Concernant les colorants de la famille des carbonyles, on peut citer par exemple les colorants choisis parmi les acridone, benzoquinone, anthraquinone, naphtoquinone, benzanthrone, anthranthrone, pyranthrone, pyrazolanthrone, pyrimidinoanthrone, flavanthrone, idanthrone, flavone, (iso)violanthrone, isoindolinone, benzimidazolone, isoquinolinone, anthrapyridone, pyrazoloquinazolone, périnone, quinacridone, quinophthalone, indigoïde, thioindigo, naphtalimide, anthrapyrimidine, dicétopyrrolopyrrole, coumarine.

Concernant les colorants de la famille des azines cycliques, on peut citer notamment les azine, xanthène, thioxanthène, fluorindine, acridine, (di)oxazine, (di)thiazine, pyronine.

Les colorants nitrés (hétéro)aromatiques sont plus particulièrement des colorants directs nitrés benzéniques ou nitrés pyridiniques.

Concernant les colorants de type porphyrines ou phtalocyanines, on peut mettre en oeuvre des composés cationiques ou non, comprenant éventuellement un ou plusieurs métaux ou ions métalliques, comme par exemple des métaux alcalins et alcalino-terreux, le zinc et le silicium.

A titre d'exemple de colorants directs particulièrement convenables, on peut citer les colorants nitrés de la série benzénique ; les colorants directs azoïques ; azométhiniques ; méthiniques ; les azacarbocyanines comme les tétraazacarbocyanines (tétraazapentaméthines) ; les colorants directs quinoniques et en particulier anthraquinoniques, naphtoquinoniques ou benzoquinoniques ; les colorants directs aziniques ; xanthéniques ; triarylméthaniques ; indoaminiques ; indigoïdes ; phtalocyanines, porphyrines et les colorants directs naturels, seuls ou en mélanges.

Ces colorants peuvent être des colorants monochromophoriques (c'est-à-dire ne comprenant qu'un seul colorant) ou polychromophoriques, de préférence di- ou tri-chromophoriques ; les chromophores pouvant être identiques ou non, de la même famille chimique ou non. A noter que qu'un colorant polychromophorique comprend plusieurs radicaux issus chacun d'une molécule absorbant dans le domaine visible entre 400 et 800 nm. De plus cette absorbance du colorant ne nécessite ni oxydation préalable de celui-ci, ni association avec d'autre(s) espèce(s) chimique(s).

Dans le cas de colorants polychromophoriques, les chromophores sont reliés entre eux au moyen d'au moins un bras de liaison qui peut être cationique ou non.

De préférence, le bras de liaison est une chaîne alkyle en C₁-C₂₀, linéaire, ramifiée ou cyclique, éventuellement interrompue par au moins un hétéroatome (tel que l'azote, l'oxygène) et/ou par au moins un groupe en comprenant (CO, SO₂), éventuellement interrompue par au moins un hétérocycle condensé ou non avec un noyau phényle et comprenant au moins un atome d'azote quaternisé engagé dans ledit cycle et éventuellement au moins un autre hétéroatome (tel que l'oxygène, l'azote ou le soufre), éventuellement interrompue par au moins un groupement phényle ou naphtyle substitué ou non, éventuellement au moins un groupement ammonium quaternaire substitué par deux groupements alkyle en C₁-C₁₅ éventuellement substitués ; le bras de liaison ne comprenant pas de groupement nitro, nitroso ou peroxo.

Si les hétérocycles ou noyaux aromatiques sont substitués, ils le sont par exemple par un ou plusieurs radicaux alkyle en C₁-C₈ éventuellement substitués par un groupement hydroxy, alcoxy en C₁-C₂, hydroxyalcoxy en C₂-C₄, acétylamino, amino substitué par un ou deux radicaux alkyle en C₁-C₄, éventuellement porteurs d'au moins un groupement hydroxyle ou les deux radicaux pouvant former avec l'atome d'azote auquel ils sont rattachés, un hétérocycle à 5 ou 6 chaînons, comprenant éventuellement un autre hétéroatome identique ou différent de l'azote ; un atome d'halogène ; un groupement hydroxyle ; un radical alcoxy en C₁-C₂ ; un radical hydroxyalcoxy en C₂-C₄; un radical amino ; un radical amino substitué par un ou deux radicaux alkyle, identiques ou différents, en C₁-C₄ éventuellement porteurs d'au moins un groupement hydroxyle.

Parmi les colorants directs benzéniques utilisables selon l'invention, on peut citer de manière non limitative les composés suivants :
- 1,4-diamino-2-nitrobenzène,
- 1-amino-2 nitro-4-β-hydroxyéthylaminobenzène
- 1-amino-2 nitro-4-bis(β-hydroxyéthyl)-aminobenzène
- 1,4-bis(β-hydroxyéthylamino)-2-nitrobenzène
- 1-β-hydroxyéthylamino-2-nitro-4-bis-(β-hydroxyéthylamino)-benzène
- 1-β-hydroxyéthylamino-2-nitro-4-aminobenzène
- 1-β-hydroxyéthylamino-2-nitro-4-(éthyl)(β-hydroxyéthyl)-aminobenzène
- 1-amino-3-méthyl-4-β-hydroxyéthylamino-6-nitrobenzène
- 1-amino-2-nitro-4-β-hydroxyéthylamino-5-chlorobenzène
- 1,2-diamino-4-nitrobenzène
- 1-amino-2-β-hydroxyéthylamino-5-nitrobenzène
- 1,2-bis-(β-hydroxyéthylamino)-4-nitrobenzène
- 1-amino-2-tris-(hydroxyméthyl)-méthylamino-5-nitrobenzène
- 1-Hydroxy-2-amino-5-nitrobenzène
- 1-Hydroxy-2-amino-4-nitrobenzène
- 1-Hydroxy-3-nitro-4-aminobenzène
- 1-Hydroxy-2-amino-4,6-dinitrobenzène
- 1-β-hydroxyéthyloxy-2-β-hydroxyéthylamino-5-nitrobenzène
- 1-Méthoxy-2-β-hydroxyéthylamino-5-nitrobenzène
- 1-β-hydroxyéthyloxy-3-méthylamino-4-nitrobenzène
- 1- β,γ-dihydroxypropyloxy-3-méthylamino-4-nitrobenzène
- 1-β-hydroxyéthylamino-4-p,γ-dihydroxypropyloxy-2-nitrobenzène
- 1-β,γ-dihydroxypropylamino-4-trifluorométhyl-2-nitrobenzène
- 1-β-hydroxyéthylamino-4-trifluorométhyl-2-nitrobenzène
- 1-β-hydroxyéthylamino-3-méthyl-2-nitrobenzène
- 1-β-aminoéthylamino-5-méthoxy-2-nitrobenzène
- 1-Hydroxy-2-chloro-6-éthylamino-4-nitrobenzène
- 1-Hydroxy-2-chloro-6-amino-4-nitrobenzène
- 1-Hydroxy-6-bis-(β-hydroxyéthyl)-amino-3-nitrobenzène
- 1-β-hydroxyéthylamino-2-nitrobenzène
- 1-Hydroxy-4-β-hydroxyéthylamino-3-nitrobenzène.

Parmi les colorants directs azoïques, azométhines, méthines ou tétraazapentaméthines utilisables selon l'invention on peut citer les colorants cationiques décrits dans les demandes de brevets WO 95/15144, WO 95/01772 et EP 714954; FR 2189006, FR 2285851, FR-2140205, EP 1378544, EP 1674073.

Ainsi, on peut tout notamment citer les colorants suivants de formules (I) à (IV), et de préférence les composés de formules (I) et (III) : 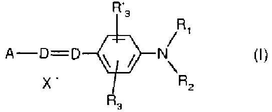 dans laquelle :
D représente un atome d'azote ou le groupement -CH,
R₁ et R₂, identiques ou différents, représentent un atome d'hydrogène ; un radical alkyle en C₁-C₄ pouvant être substitué par un radical -CN, -OH ou -NH₂ ou forment avec un atome de carbone du cycle benzénique un hétérocycle éventuellement oxygéné ou azoté, pouvant être substitué par un ou plusieurs radicaux alkyle en C₁-C₄ un radical 4'-aminophényle,
R₃ et R'₃, identiques ou différents, représentent un atome d'hydrogène ou d'halogène choisi parmi le chlore, le brome, l'iode et le fluor, un radical cyano, alkyl en C₁-C₄, alcoxy en C₁-C₄ ou acétyloxy,
X - représente un anion de préférence choisi parmi le chlorure, le méthyl sulfate et l'acétate,
A représente un groupement choisi par les structures A1 à A18 suivantes : 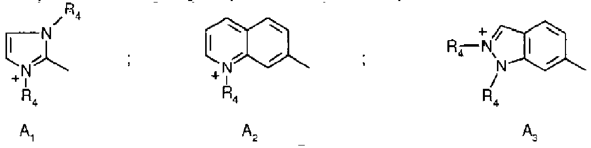 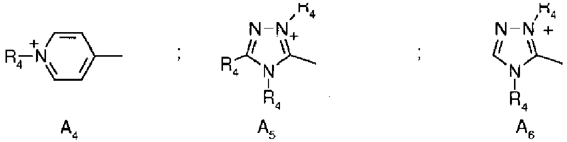 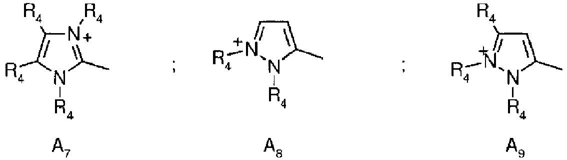 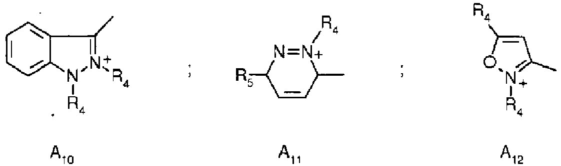 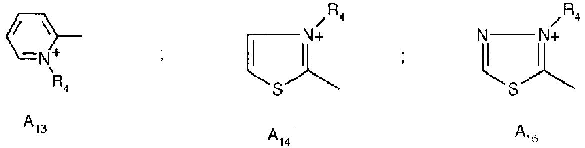 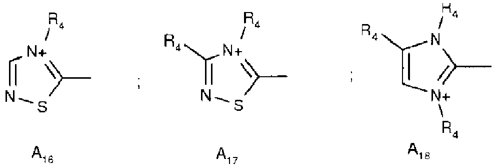
dans lesquelles R₄ représente un radical alkyle en C₁-C₄ pouvant être substitué par un radical hydroxyle et R₅ représente un radical alcoxy en C₁-C₄ ; 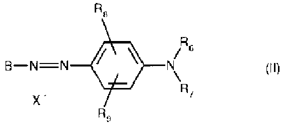 dans laquelle :
R₆ représente un atome d'hydrogène ou un radical alkyle en C₁-C₄,
R₇ représente un atome d'hydrogène, un radical alkyle pouvant être substitué par un radical -CN ou par un groupement amino, un radical 4'-aminophényle ou forme avec R₆ un hétérocycle éventuellement oxygéné et/ou azoté pouvant être substitué par un radical alkyle en C₁-C₄,
R₈ et R₉, identiques ou différents, représentent un atome d'hydrogène, un atome d'halogène tel que le brome, le chlore, l'iode ou le fluor, un radical alkyle en C₁-C₄ ou alcoxy en C₁-C₄, un radical -CN,
X - représente un anion de préférence choisi parmi le chlorure, le méthyl sulfate et l'acétate,
B représente un groupement choisi par les structures B1 à B6 suivantes 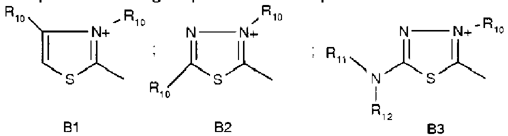 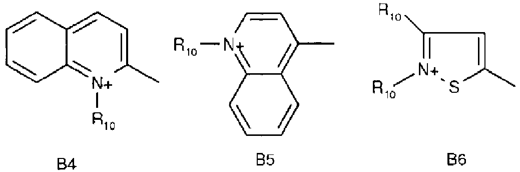
dans lesquelles R₁₀ représente un radical alkyle en C₁-C₄, R₁₁ et R₁₂, identiques ou différents, représentent un atome d'hydrogène ou un radical alkyle en C₁-C₄ ; 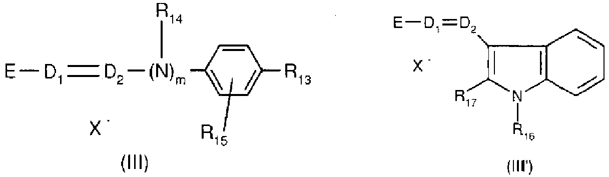 dans lesquelles :
R₁₃ représente un atome d'hydrogène, un radical alcoxy en C₁-C₄, un atome d'halogène tel que le brome, le chlore, l'iode ou le fluor,
R₁₄ représente un atome d'hydrogène, un radical alkyle en C₁-C₄ ou forme avec un atome de carbone du cycle benzénique un hétérocycle éventuellement oxygéné et/ou substitué par un ou plusieurs groupements alkyle en C₁-C₄,
R₁₅ représente un atome d'hydrogène ou d'halogène tel que le brome, le chlore, l'iode ou le fluor,
R₁₆ et R₁₇, identiques ou différents, représentent un atome d'hydrogène ou un radical alkyle en C₁-C₄,
D₁ et D₂, identiques ou différents, représentent un atome d'azote ou le groupement -CH, m = 0 ou 1, étant entendu que lorsque R₁₃ représente un groupement amino non substitué, alors D₁ et D₂ représentent simultanément un groupement -CH et m = 0,
X - représente un anion de préférence choisi parmi le chlorure, le méthyl sulfate et l'acétate,
E représente un groupement choisi par les structures E1 à E8 suivantes 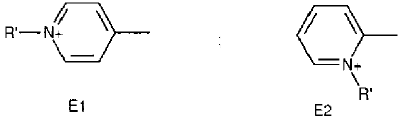 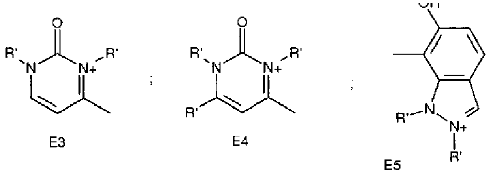 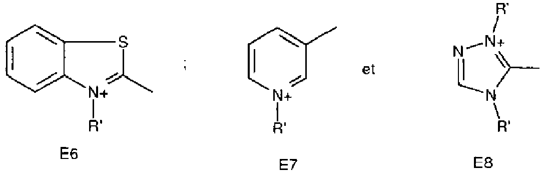
dans lesquelles R' représente un radical alkyle en C₁-C₄ ; lorsque m = 0 et que D₁ représente un atome d'azote, alors E peut également désigner un groupement de structure E9 suivante: 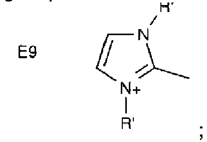 dans laquelle R' représente un radical alkyle en C₁-C₄.

G-N=N-J (IV)

dans laquelle :
le symbole G représente un groupement choisi parmi les structures G₁ à G₃ suivantes : 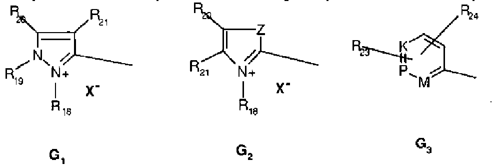 structures G₁ à G₃ dans lesquelles,
R₁₈ désigne un radical alkyle en C₁-C₄, un radical phényle pouvant être substitué par un radical alkyle en C₁-C₄ ou un atome d'halogène choisi parmi le chlore, le brome, l'iode et le fluor ;
R₁₉ désigne un radical alkyle en C₁-C₄ ou un radical phényle;
R₂₀ et R₂₁, identiques ou différents, représentent un radical alkyle en C₁-C₄, un radical phényle, ou forment ensemble dans G₁ un cycle benzénique substitué par un ou plusieurs radicaux alkyle en C₁-C₄, alcoxy en C₁-C₄, ou NO₂ ou forment ensemble dans G₂ un cycle benzénique éventuellement substitué par un ou plusieurs radicaux alkyle en C₁-C₄ , alcoxy en C₁-C₄, ou NO₂
R₂₀ peut désigner en outre un atome d'hydrogène;
Z désigne un atome d'oxygène, de soufre ou un groupement -NR₁₉;
M représente un groupement -CH, -CR (R désignant alkyle en C₁-C₄), ou -NR2₂(X-)ᵣ;
K représente un groupement -CH, -CR (R désignant alkyle en C₁-C₄), ou -NR2₂(X-)ᵣ;
P représente un groupement -CH, -CR (R désignant alkyle en C₁-C₄), ou -NR2₂(X⁻)ᵣ; r désigne zéro ou 1 ;
R₂₂ représente un atome O⁻, un radical alcoxy en C₁-C₄, ou un radical alkyle en C₁-C₄;
R₂₃ et R₂₄, identiques ou différents, représentent un atome d'hydrogène ou d'halogène choisi parmi le chlore, le brome, l'iode et le fluor, un radical alkyle en C₁-C₄, alcoxy en C₁-C₄, un radical -NO₂
X⁻ représente un anion de préférence choisi parmi le chlorure, l'iodure, le méthyl sulfate, l'éthyl sulfate, l'acétate et le perchlorate; sous réserve que,
si R₂₂ désigne O⁻, alors r désigne zéro;
si K ou P ou M désignent -N-alkyle C₁-C₄ X⁻, alors R₂₃ ou R₂₄ est ou non différent d'un atome d'hydrogène;
si K désigne -NR₂₂(X⁻)ᵣ , alors M= P= -CH, -CR;
si M désigne -NR₂₂(X⁻)ᵣ , alors K= P= -CH, -CR;
si P désigne -NR₂₂(X⁻)ᵣ , alors K= M et désignent -CH ou -CR;
si Z désigne un atome de soufre avec R₂₁ désignant alkyle en C₁-C₄ , alors R₂₀ est différent d'un atome d'hydrogène;
si Z désigne -NR₂₂ avec R₁₉ désignant alkyle en C₁-C₄ , alors au moins l'un des radicaux R₁₈, R₂₀ ou R₂₁ du groupement de structure G₂ est différent d'un radical alkyle en C₁-C₄;
le symbole J représente :
   - (a) un groupement de structure J₁ suivante 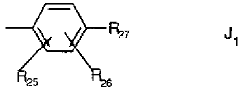
      structure J₁ dans laquelle, R₂₅ représente un atome d'hydrogène, un atome d'halogène choisi parmi le chlore, le brome, l'iode et le fluor, un radical alkyle en C₁-C₄, alcoxy en C₁-C₄, un radical -OH, -NO₂, -NHR₂₈, -NR₂₉R₃₀, -NHCOalkyle en C₁-C₄, ou forme avec R₂₆ un cycle à 5 ou 6 chaînons contenant ou non un ou plusieurs hétéroatomes choisis parmi l'azote, l'oxygène ou le soufre;
      R₂₆ représente un atome d'hydrogène, un atome d'halogène choisi parmi le chlore, le brome, l'iode et le fluor, un radical alkyle en C₁-C₄, alcoxy en C₁-C₄, ou forme avec R₂₇ ou R₂₈ un cycle à 5 ou 6 chaînons contenant ou non un ou plusieurs hétéroatomes choisis parmi l'azote, l'oxygène ou le soufre; R₂₇ représente un atome d'hydrogène, un radical -OH, un radical -NHR₂₈, un radical - NR₂₉R₃₀;
      R₂₈ représente un atome d'hydrogène, un radical alkyle en C₁-C₄, un radical monohydroxyalkyle en C₁-C₄, polyhydroxyalkyle en C₂-C₄, un radical phényle; R₂₉ et R₃₀, identiques ou différents, représentent un radical alkyle en C₁-C₄ , un radical monohydroxyalkyle en C₁-C₄, polyhydroxyalkyle en C₂-C₄;
   - (b) un groupement hétérocyclique azoté à 5 ou 6 chaînons susceptible de renfermer d'autres hétéroatomes et/ou des groupements carbonylés et pouvant être substitué par un ou plusieurs radicaux alkyle en C₁-C₄, amino ou phényle, et notamment un groupement de structure J₂ suivante 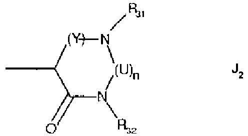 structure J₂ dans laquelle, R₃₁ et R₃₂, identiques ou différents, représentent un atome d'hydrogène, un radical alkyle en C₁-C₄, un radical phényle; Y désigne le radical -CO- ou le radical 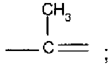 n = 0 ou 1, avec, lorsque n désigne 1, U désigne le radical -CO- .

Dans les structures (I) à (IV) définies ci-dessus le groupement alkyle ou alcoxy en C₁-C₄ désigne de préférence méthyle, éthyle, butyle, méthoxy, éthoxy.

Parmi les composés de formules (I) et (III), on préfère les composés suivants :

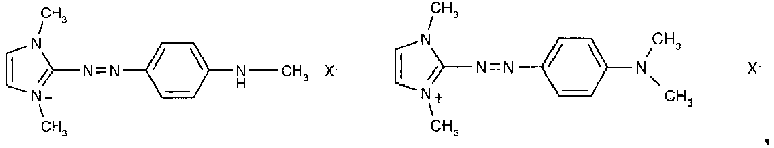

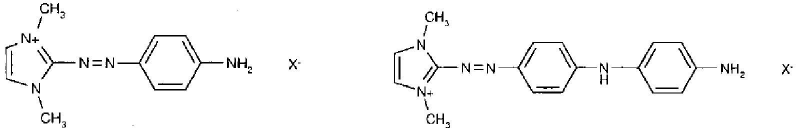

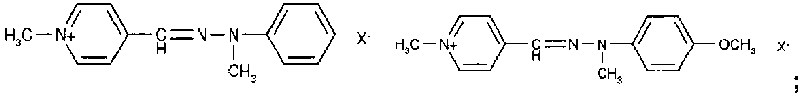

On peut également citer parmi les colorants directs azoïques les colorants suivants, décrits dans le COLOUR INDEX INTERNATIONAL 3e édition :
- Disperse Red 17
- Basic Red 22
- Basic Red 76
- Basic Yellow 57
- Basic Brown 16
- Basic Brown 17
- Disperse Black 9.

On peut aussi citer le 1-(4'-aminodiphénylazo)-2-méthyl-4bis-(β-hydroxyéthyl) aminobenzène.

Parmi les colorants directs quinoniques on peut citer les colorants suivants :
- Disperse Red 15
- Solvent Violet 13
- Disperse Violet 1
- Disperse Violet 4
- Disperse Blue 1
- Disperse Violet 8
- Disperse Blue 3
- Disperse Red 11
- Disperse Blue 7
- Basic Blue 22
- Disperse Violet 15
- Basic Blue 99
ainsi que les composés suivants :
- 1-N-méthylmorpholiniumpropylamino-4-hydroxyanthraquinone
- 1-Aminopropylamino-4-méthylaminoanthraquinone
- 1-Aminopropylaminoanthraquinone
- 5-β-hydroxyéthyl-1,4-diaminoanthraquinone
- 2-Aminoéthylaminoanthraquinone
- 1,4-Bis-(β,γ-dihydroxypropylamino)-anthraquinone.

Parmi les colorants aziniques, on peut citer les composés suivants :
- Basic Blue 17
- Basic Red 2.

Parmi les colorants triarylméthaniques utilisables selon l'invention, on peut citer les composés suivants :
- Basic Green 1
- Basic Violet 3
- Basic Violet 14
- Basic Blue 7
- Basic Blue 26

Parmi les colorants indoaminiques utilisables selon l'invention, on peut citer les composés suivants :
- 2-β-hydroxyéthlyamino-5-[bis-(β-4'-hydroxyéthyl)amino]anilino-1,4-benzoquinone
- 2-β-hydroxyéthylamino-5-(2'-méthoxy-4'-amino)anilino-1,4-benzoquinone
- 3-N(2'-Chloro-4'-hydroxy)phényl-acétylamino-6-méthoxy-1,4-benzoquinone imine
- 3-N(3'-Chloro-4'-méthylamino)phényl-uréido-6-méthyl-1,4-benzoquinone imine
- 3-[4'-N-(Ethyl,carbamylméthyl)-amino]-phényl-uréido-6-méthyl-1,4-benzoquinone imine.

Parmi les colorants de type tétraazapentaméthiniques utilisables selon l'invention, on peut citer les composés suivants figurant dans le tableau ci-dessous:

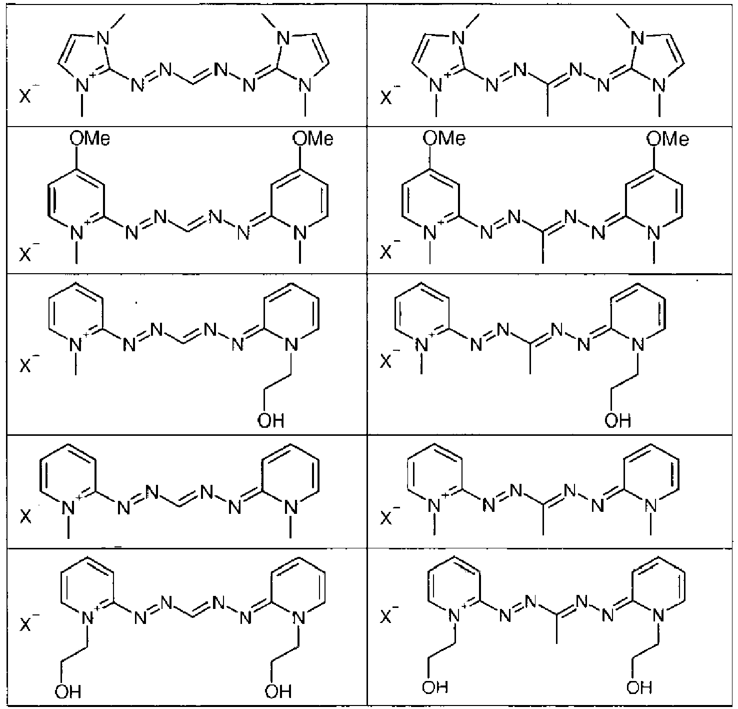

X⁻ représente un anion de préférence choisi parmi le chlorure, l'iodure, le méthyl sulfate, l'éthyl sulfate, l'acétate et le perchlorate.

Parmi les colorants polychromophoriques, on peut citer plus particulièrement les colorants di- ou tri- chromophoriques azoïques et/ou azométhiniques (hydrazoniques), symétriques ou non, comprenant d'une part au moins un hétérocycle aromatique comprenant 5 ou 6 chaînons, éventuellement condensé, comprenant au moins un atome d'azote quaternisé engagé dans ledit hétérocycle et éventuellement au moins un autre hétéroatome (tel que l'azote, le soufre, l'oxygène), et d'autre part, au moins un groupement phényle ou naphtyle, éventuellement substitué, éventuellement porteur d'au moins un groupement OR avec R représentant un atome d'hydrogène, un radical alkyle éventuellement substitué en C₁-C₆, un noyau phényle éventuellement substitué, ou d'au moins un groupement N(R')₂ avec R' identiques ou non, représentant un atome d'hydrogène, un radical alkyle éventuellement substitué en C₁-C₆, un noyau phényle éventuellement substitué ; les radicaux R' pouvant former avec l'atome d'azote auquel ils sont liés, un hétérocycle saturé à 5 ou 6 chaînons, ou bien encore l'un et/ou les deux radicaux R' peuvent former chacun avec l'atome de carbone du cycle aromatique placé en ortho de l'atome d'azote, un hétérocycle saturé à 5 ou 6 chaînons.

A titre d'hétérocycle cationique aromatique, on peut citer de préférence, les cycles à 5 ou 6 chaînons comprenant 1 à 3 atomes d'azote, de préférence 1 ou 2 atomes d'azote, l'un étant quaternisé ; ledit hétérocycle étant par ailleurs éventuellement condensé à un noyau benzénique. Il est à noter de même que l'hétérocycle peut éventuellement comprendre un autre hétéroatome différent de l'azote, comme le soufre ou l'oxygène.

Si les hétérocycles ou groupements phényle ou napthyle sont substitués, ils le sont par exemple par un ou plusieurs radicaux alkyle en C₁-C₈ éventuellement substitués par un groupement hydroxy, alcoxy en C₁-C₂, hydroxyalcoxy en C₂-C₄, acétylamino, amino substitué par un ou deux radicaux alkyle en C₁-C₄, éventuellement porteurs d'au moins un groupement hydroxyle ou les deux radicaux pouvant former avec l'atome d'azote auquel ils sont rattachés, un hétérocycle à 5 ou 6 chaînons, comprenant éventuellement un autre hétéroatome identique ou différent de l'azote ; un atome d'halogène ; un groupement hydroxyle ; un radical alcoxy en C₁-C₂; un radical hydroxyalcoxy en C₂-C₄; un radical amino ; un radical amino substitué par un ou deux radicaux alkyle, identiques ou différents, en C₁-C₄ éventuellement porteurs d'au moins un groupement hydroxyle.

Ces polychromophores sont reliés entre eux au moyen d'au moins un bras de liaison comprenant éventuellement au moins un atome d'azote quaternisé engagé ou non dans un hétérocycle saturé ou non, éventuellement aromatique.

De préférence, le bras de liaison est une chaîne alkyle en C₁-C₂₀, linéaire, ramifiée ou cyclique, éventuellement interrompue par au moins un hétéroatome (tel que l'azote, l'oxygène) et/ou par au moins un groupe en comprenant (CO, SO₂), éventuellement interrompue par au moins un hétérocycle condensé ou non avec un noyau phényle et comprenant au moins un atome d'azote quaternisé engagé dans ledit cycle et éventuellement au moins un autre hétéroatome (tel que l'oxygène, l'azote ou le soufre), éventuellement interrompue par au moins un groupement phényle ou naphtyle substitué ou non, éventuellement au moins un groupement ammonium quaternaire substitué par deux groupements alkyle en C₁-C₁₅ éventuellement substitués ; le bras de liaison ne comprenant pas de groupement nitro, nitroso ou peroxo.

La liaison entre le bras de liaison et chaque chromophore se fait en général au moyen d'un hétéroatome substituant le noyau phényle ou napthyle ou au moyen de l'atome d'azote quaternisé de l'hétérocycle cationique.

Le colorant peut comprendre des chromophores identiques ou non.

A titre d'exemples de tels colorants, on pourra notamment se reporter aux demandes de brevets EP 1637566, EP 1619221, EP 1634926, EP 1619220, EP 1672033, EP 1671954, EP 1671955, EP 1679312, EP 1671951, EP167952, EP167971, WO 06/063866, WO 06/063867, WO 06/063868, WO 06/063869, EP 1408919, EP 1377264, EP 1377262, EP 1377261, EP 1377263, EP 1399425, EP 1399117, EP 1416909, EP 1399116, EP 1671560.

On peut aussi également mettre en oeuvre des colorants directs cationiques cités dans les demandes EP 1006153, qui décrit des colorants comprenant deux chromophores de type anthraquinones reliés au moyen d'un bras de liaison cationique ; EP 1433472, EP 1433474, EP 1433471 et EP 1433473 qui décrivent des colorants dichromophoriques identiques ou non, reliés par un bras de liaison cationique ou non, ainsi que EP 6291333 qui décrit notamment des colorants comprenant trois chromophores, l'un d'eux étant un chromophore anthraquinone auquel sont reliés deux chromophores de type azoïque ou diazacarbocyanine ou l'un de ses isomères.

Parmi les colorants directs naturels utilisables selon l'invention, on peut citer la lawsone, la juglone, l'alizarine, la purpurine, l'acide carminique, l'acide kermésique, la purpurogalline, le protocatéchaldéhyde, l'indigo, l'isatine, la curcumine, la spinulosine, l'apigénidine, les orcéines. On peut également utiliser les extraits ou décoctions contenant ces colorants naturels et notamment les cataplasmes ou extraits à base de henné.

Lorsqu'ils sont présents, le ou les colorants directs représentent plus particulièrement de 0,0001 à 10 % en poids du poids total de la composition, et de préférence de 0,005 à 5 % en poids.

La composition (C) peut comprendre l'un et ou l'autre types de colorants. Elle peut éventuellement correspondre à deux compositions colorantes l'une comprenant le ou les colorants d'oxydation, l'autre, le ou les colorants directs.

Un autre ingrédient de la composition (C) est représenté par une ou plusieurs amines organiques dont le pKb à 25°C est inférieur à 12, de préférence inférieur à 10, et encore plus préférentiellement inférieur à 6.

Il est à noter qu'il s'agit du pKb correspondant à la fonction de basicité la plus élevée.

Selon une première variante de l'invention, l'amine organique comprend une ou deux fonctions amine primaire, secondaire ou tertiaire, et un ou plusieurs groupements alkyle, linéaires ou ramifiés, en C₁-C₈ porteurs d'un ou plusieurs radicaux hydroxyle.

Conviennent en particulier à la réalisation de l'invention les amines organiques choisies parmi les alcanolamines telles que les mono-, di- ou tri- alcanolamines, comprenant un à trois radicaux hydroxyalkyle, identiques ou non, en C₁-C₄.

Parmi des composés de ce type, on peut citer la monoéthanolamine, la diéthanolamine, la triéthanolamine, la monoisopropanolamine, la diisopropanolamine, la N-diméthylaminoéthanolamine, le 2-amino-2-méthyl-1-propanol, la triisopropanolamine, le 2-amino-2-méthyl-1,3-propanediol, le 3-amino-1,2-propanediol, le 3-diméthylamino-1,2-propanediol, le tris-hydroxyméthylaminométhane.

Conviennent également les amines organiques de formule suivante 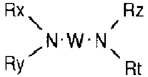 dans laquelle W est un reste alkylène en C₁-C₆ éventuellement substitué par un groupement hydroxyle ou un radical alkyle en C₁-C₆ ; Rx, Ry, Rz et Rt, identiques ou différents, représentent un atome d'hydrogène, un radical alkyle en C₁-C₆ ou hydroxyalkyle en C₁-C₆, aminoalkyle en C₁-C₆.

On peut citer à titre d'exemple de telles amines, le 1,3 diaminopropane, le 1,3 diamino 2 propanol, la spermine, la spermidine.

Selon une autre variante de l'invention, l'amine organique est choisie parmi les acides aminés.

Plus particulièrement, les acides aminés utilisables sont d'origine naturelle ou de synthèse, sous leur forme L, D, ou racémique et comportent au moins une fonction acide choisie plus particulièrement parmi les fonctions acides carboxyliques, sulfoniques, phosphoniques ou phosphoriques. Les acides aminés peuvent se trouver sous forme neutre ou ionique.

De manière avantageuse, les acides aminés sont des acides aminés basiques comprenant une fonction amine supplémentaire éventuellement incluse dans un cycle ou dans une fonction uréido.

De tels acides aminés basiques sont choisis de préférence parmi ceux répondant à la formule (I) suivante: 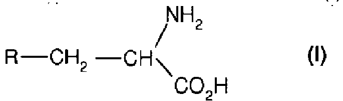 où R désigne un groupe choisi parmi

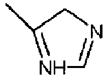

-(CH₂)₃NH₂ ;

-(CH₂)₂NH₂ ;

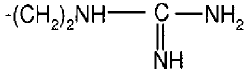

-(CH₂)₂NHCONH₂ ;

Les composés correspondants à la formule (I) sont l'histidine, la lysine, l'arginine, l'ornithine, la citrulline.

A titre d'acides aminés utilisables dans la présente invention, on peut notamment citer l'acide aspartique, l'acide glutamique, l'alanine, l'arginine, l'ornithine, la citrulline, l'asparagine, la carnitine, la cystéine, la glutamine, la glycine, l'histidine, la lysine, l'isoleucine, la leucine, la méthionine, la N-phénylalanine, la proline, la serine, la taurine la thréonine, le tryptophane, la tyrosine et la valine.

Le ou les acides aminés, peuvent être mis en oeuvre en mélange avec un ou plusieurs adjuvants solides ou pâteux, et de préférence pulvérulents. Les adjuvants peuvent être choisis parmi les argiles, les sels, les tensioactifs anioniques, non ioniques, cationiques ou zwittérioniques, les épaississants naturels ou de synthèse, l'amidon éventuellement modifié, les billes de verre, la silice, le nylon, l'alumine, le dioxyde de titane, les zéolithes, le poly(méthacrylate de méthyle) (PMMA), le chitosane, la maltodextrine, la cyclodextrine, les mono- ou disaccharides comme le glucose, le saccharose, le sorbitol ou le fructose, l'oxyde de zinc, de zirconium, les silicabades, le talc, les borosilicates notamment de calcium, le polyéthylène, le polytétrafluoroéthylène (PTFE), la cellulose et ses dérivés, les composés superabsorbants, les carbonates de magnésium ou de calcium, le polyacrylamide, l'hydroxyapatite poreux, la sciure de bois, la poudre de fucus, la polyvinylpyrrolidone réticulée, l'alginate de calcium, le charbon actif, les particules de poly(chlorure de vinylidène/acrylonitrile), notamment celles commercialisées sous la dénomination générale d'« Expancel® » par la société AKZO NOBEL sous les références particulières « Expancel® WE» ou « DE » Expancels, et leurs mélanges.

Selon une variante préférée de l'invention, l'amine organique est choisie parmi les acides aminés basiques. Les acides aminés particulièrement préférés sont l'arginine, la lysine, l'histidine, ou leurs mélanges.

Selon une autre variante de l'invention, l'amine organique est choisie parmi les amines organiques de type hétérocycliques On peut en particulier citer, outre l'histidine déjà mentionnée dans les acides aminés, la pyridine, la pipéridine, l' imidazole, le triazole, le tétrazole, le benzimidazole.

Selon une autre variante de l'invention, l'amine organique est choisie parmi les dipeptides d'acides aminés. A titre de dipeptides d'acides aminés utilisables dans la présente invention, on peut notamment citer la carnosine, l'anserine et la baleine

Selon une autre variante de l'invention, l'amine organique est choisie parmi les composés comportant une fonction guanidine. A titre d'amines d'amines de ce type utilisables dans la présente invention, on peut notamment citer outre l'arginine déjà mentionnée à titre d'acide aminé, la créatine, la créatinine, la 1,1-diméthylguanidine, 1,1-diéthylguanidine, la glycocyamine, la metformin, l'agmatine, la n-amidinoalanine, l'acide 3-guanidinopropionique, l'acide 4-guanidinobutyrique et l'acide 2-([amino(imino)methyl]amino)ethane-1-sulfonique.

De préférence l'amine organique présente dans la composition anhydre est une alcanolamine. Plus préférentiellement l'amine organique est choisie parmi le 2-amino 2-méthyl 1-propanol, la monoéthanolamine ou leurs mélanges. Encore plus préférentiellement l'amine organique est la monoéthanolamine.

De manière avantageuse, la composition (C) présente une teneur en amine organique allant de 0,1 à 50 % en poids, de préférence de 0,5 à 20 % en poids par rapport au poids de ladite composition.

La composition (C) peut être une composition anhydre ou aqueuse. Par composition aqueuse, on entend une composition comprenant plus de 5 % en poids d'eau, de préférence plus de 10 % en poids d'eau, et de manière encore plus avantageuse plus de 20 % en poids d'eau.

De préférence, la composition (C) est une composition aqueuse.

La composition peut éventuellement comprendre un ou plusieurs solvants. Ceux qui ont été cités dans le cadre de la description de la composition aqueuse (B) peuvent convenir à la composition (C), à hauteur des concentrations précisées également.

La composition (C) peut également comprendre des additifs classiques tels que ceux qui ont été listés auparavant et l'on pourra s'y reporter.

Le pH de la composition (C) si elle est aqueuse est compris entre 2 et 12, de préférence entre 8 et 11. Le pH est adapté en utilisant des agents acidifiants ou alcalinisants, tels que ceux mentionnés précédemment.

Conformément auprocédé selon l'invention, on applique sur les fibres kératiniques, sèches ou humides, une composition obtenue par mélange extemporané avant l'application, des compositions (A), (B) et (C).

Selon ce procédé les rapports pondéraux R1 des quantités de compositions (A) + (C) / (B) et R2 des quantités de compositions (A) / (C) varient de 0,1 à 10, et de préférence de 0,3 à 3.

Selon un mode préféré, la composition aqueuse oxydante (B) représente 50% à 70% du poids total du mélange des compositions (A), (B) et (C) à appliquer sur les cheveux.

En outre, le mélange présent sur les fibres (résultant soit du mélange extemporané des compositions soit de l'application successive de celles-ci) est laissé en place pour une durée, en général, de l'ordre de 1 minute à 1 heure, de préférence de 5 minutes à 30 minutes.

La température durant le procédé est classiquement comprise entre la température ambiante (entre 15 à 25°C) et 80°C, de préférence entre la température ambiante et 60°C.

A l'issue du traitement, les fibres kératiniques humaines sont éventuellement rincées à l'eau, subissent éventuellement un lavage avec un shampooing suivi d'un rinçage à l'eau, avant d'être séchées ou laissées à sécher.

Il est précisé que si la composition appliquée sur les cheveux (comprenant les compositions (A), (B) et (C)) comprenait de l'ammoniaque, sa teneur serait de préférence inférieure ou égale à 0,03 % en poids de la composition finale (exprimée en NH₃), plus particulièrement inférieure ou égale à 0,01 % en poids par rapport à la composition finale. Il est indiqué que la composition finale résulte du mélange des compositions (A), (B) et (C) ; ces mélanges étant réalisés avant application sur les fibres kératiniques (préparation extemporanée)

Mais les compositions (A), (B) et (C) ne comprennent de préférence pas d'ammoniaque.

Les exemples suivants servent à illustrer l'invention sans toutefois présenter un caractère limitatif.

### EXEMPLE 1

On prépare les compositions suivantes (quantités exprimées en grammes):

| Composition (A) | |
|---|---|
| Mono-laurate de sorbitane oxyéthyléné (4OE) | 21,67 |
| Silice pyrogénée à caractère hydrophobe | 11 |
| Huile vaseline | Qsp 100 |

| Composition (C) | |
|---|---|
| Paraphénylène diamine | 6,55 |
| Résorcinol | 4,95 |
| 2-méthyl-résorcinol | 1,86 |
| 2,4-diaminophenoxyéthanol HCI | 0,15 |
| Métabisulfite de sodium | 0,455 |
| Acide érythorbique | 0,31 |
| Monoéthanolamine pure | 40,07 |
| Eau | Qsp 100 |

Au moment de l'emploi, on mélange :
- 9 parties en poids de la composition (A)
- 1 partie en poids de la composition (C)
- 10 parties en poids d'une composition aqueuse oxydante comprenant 6 % de peroxyde d'hydrogène à pH de 2,3 et comprenant environ 80% d'eau.

Le mélange obtenu, dont le pH est environ 10, est ensuite appliqué sur une mèche de cheveu naturelle à 90% blancs (BN) et sur une mèche BN permanentée (BP).

Le temps de pause est de 30 minutes à température ambiante.

A l'issue de ce temps, les mèches sont rincées, puis lavées avec du shampooing Elsève multivitamines.

Comme le montre le tableau ci-dessous, la composition de l'invention conduit à une coloration mate, puissante et peu sélective.

| | L* | a* | b* | Sélectivité |
|---|---|---|---|---|
| BN | 20.37 | 2.53 | 3.83 | 3.18 |
| BP | 17.78 | 1.68 | 2.19 | |

### EXEMPLE 2

On prépare les compositions suivantes :

**Composition (A) (identique à l'exemple 1)**

| Composition (C) | |
|---|---|
| 1-hydroxy-4-amino-benzène | 3,27 |
| 2-amino-3-hydroxypiridine | 3,30 |
| Métabisulfite de sodium | 0,455 |
| Acide érythorbique | 0,31 |
| Monoéthanolamine pure | 40,0 |
| Alcool oléique polyglycérolé à 2 moles de glycérol | 2,1 |
| Alcool oléique polyglycérolé à 4 moles de glycérol | 3,0 MA |
| Acide oléique | 1,58 |
| Amine oléique à 2 moles d'oxyde d'éthylène (ETHOMEEN 012 / Akzo) | 3,69 |
| Laurylamino succinamate de diéthylaminopropyle, sel de sodium (55% en matière active) | 1,58 MA |
| Alcool oléique | 2,64 |
| Diethanolamide d'acide oléique | 6,32 |
| Alcool éthylique | 3,69 |
| Propylène glycol | 1,84 |
| Dipropylèneglycol | 0,26 |
| Monométhylether de propylèneglycol | 4,74 |
| Eau déminéralisée q.s.p. | Qsp 100 |

On effectue la coloration dans les mêmes conditions que celles détaillées dans l'exemple 1.

Comme le montre le tableau ci-dessous, la composition de l'invention conduit à un cuivré puissant et peu sélectif.

| | L* | a* | b* | Sélectivité |
|---|---|---|---|---|
| BN | 42,23 | 19,29 | 29,42 | 2,09 |
| BP | 42,47 | 21,34 | 29,73 | |

## Revendications

1. Procédé de coloration de fibres kératiniques humaines en présence d'un agent oxydant dans lequel on applique sur lesdites fibres :
(a) une composition anhydre (A) cosmétique comprenant un ou plusieurs corps gras, le ou les corps gras étant choisis parmi l'huile de vaseline, les polydécènes ou leurs mélanges, la teneur en corps en corps gras étant comprise entre 10 et 99 % en poids, par rapport au poids de la composition (A), et un ou plusieurs tensioactifs ;
(b) une composition (B) comprenant un ou plusieurs agents oxydants ;
(c) une composition (C) comprenant un ou plusieurs colorants d'oxydation, un ou plusieurs colorants directs, ou leurs mélanges une ou plusieurs amines organiques dont le pKb est inférieur à 12 à 25°C,
**caractérisé en ce que** l'on applique une composition obtenue par mélange extemporané avant application, des compositions (A), (B) et (C).

2. Procédé selon l'une quelconque des revendications précédentes, **caractérisé en ce que** le tensioactif présent dans la composition (A) est un tensioactif non ionique, plus particulièrement choisi parmi les tensioactifs non ioniques mono- ou polyoxyalkylénés, mono- ou poly- glycérolés.

3. Procédé selon l'une quelconque des revendications précédentes, **caractérisé en ce que** la teneur en tensioactifs représente de 0,1 à 50 % en poids, par rapport au poids de la composition anhydre (A).

4. Procédé selon l'une quelconque des revendications précédentes, **caractérisé en ce que** l'amine organique comprend une ou deux fonctions amine primaire, secondaire ou tertiaire, et un ou plusieurs groupements alkyle, linéaires ou ramifiés, en C₁-C₈ porteurs d'un ou plusieurs radicaux hydroxyle.

5. Procédé selon la revendication précédente, **caractérisé en ce que** l'amine organique est :
• une alcanolamine choisie parmi les mono-, di- ou tri- alcanolamine, comprenant un à trois radicaux hydroxyalkyle, identiques ou non, en C₁-C₄,
• un composé de formule suivante : 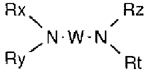 dans laquelle W est un reste alkylène en C₁-C₆ éventuellement substitué par un groupement hydroxyle ; Rx, Ry, Rz et Rt, identiques ou différents, représentent un atome d'hydrogène, un radical alkyle en C₁-C₆ ou hydroxyalkyle en C₁-C₆, un radical aminoalkyle en C₁-C₆.

6. Procédé selon l'une quelconque des revendications précédentes, **caractérisé en ce que** l'amine organique est une alcanolamine, de préférence choisie parmi le 2-amino 2-méthyl 1-propanol, la monoéthanolamine ou leurs mélanges.

7. Procédé selon l'une quelconque des revendications précédentes, **caractérisé en ce que** l'amine organique est choisie parmi les acides aminés basiques de formule (1) suivante : 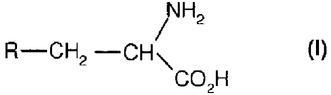 où R désigne un groupe choisi parmi : 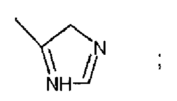 -(CH₂)₃NH₂ ; -(CH₂)₂NH₂ ; -(CH₂)₂NHCONH₂ ;

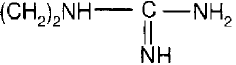

8. Procédé selon l'une quelconque des revendications précédentes, **caractérisé en ce que** l'amine organique est choisie parmi l'arginine, l'histidine, la lysine, ou leurs mélanges.

9. Procédé selon l'une quelconque des revendications précédentes, **caractérisé en ce que** la teneur en amine organique représente de 0,1 à 40 % en poids, de préférence de 0,5 à 20 % en poids par rapport au poids de la composition (B).

10. Procédé selon l'une quelconque des revendications précédentes, **caractérisé en ce que** les rapports pondéraux R1 des quantités de compositions (A) + (C) / (B) et R2 des quantités de compositions (A) / (C) varient de 0,1 à 10, et de préférence de 0,3 à 3.

## Patentansprüche

1. Verfahren zum Färben von menschlichen Keratinfasern in Gegenwart eines Oxidationsmittels, bei dem man auf die Fasern
(a) eine wasserfreie kosmetische Zusammensetzung (A), die eine oder mehrere Fettsubstanzen und ein oder mehrere Tenside umfasst, **dadurch gekennzeichnet, dass** die Fettsubstanz bzw. Fettsubstanzen aus Vaselineöl, Polydecenen, flüssigen Estern oder Mischungen davon ausgewählt ist bzw. sind, und **dadurch gekennzeichnet, dass** der Gehalt an Fettsubstanz zwischen 10 und 99 Gew.-%, bezogen auf das Gewicht der Zusammensetzung (A), liegt;
(b) eine Zusammensetzung (B), die ein oder mehrere Oxidationsmittel umfasst;
(c) eine Zusammensetzung (C), die einen oder mehrere Oxidationsfarbstoffe, einen oder mehrere Direktfarbstoffe oder Mischungen davon und ein oder mehrere organische Amine mit einem pKb-Wert von weniger als 12 bei 25°C umfasst;
Aufbringt,
**dadurch gekennzeichnet, dass** man eine durch unmittelbares Mischen der Zusammensetzungen (A), (B) und (C) und vor dem Aufbringen erhaltene Mischung aufbringt.

2. Verfahren nach einem der vorhergehenden Ansprüche, **dadurch gekennzeichnet, dass** es sich bei dem in der Zusammensetzung (A) vorliegenden Tensid um ein nichtionisches Tensid, das insbesondere aus mono- oder polyoxyalkylenierten, mono- oder polyglycerinierten nichtionischen Tensiden ausgewählt ist, handelt.

3. Verfahren nach einem der vorhergehenden Ansprüche, **dadurch gekennzeichnet, dass** der Gehalt an Tensiden 0,1 bis 50 Gew.-%, bezogen auf das Gewicht der wasserfreien Zusammensetzung (A), ausmacht.

4. Verfahren nach einem der vorhergehenden Ansprüche, **dadurch gekennzeichnet, dass** das organische Amin eine oder zwei primäre, sekundäre oder tertiäre Aminfunktionen und eine oder mehrere lineare oder verzweigte C₁-C₈-Alkylgruppen mit einem oder mehreren Hydroxylresten umfasst.

5. Verfahren nach dem vorhergehenden Anspruch, **dadurch gekennzeichnet, dass** es sich bei dem organischen Amin um
• ein Alkanolamin, ausgewählt aus Mono-, Di- oder Trialkanolaminen, mit einem bis drei gleichen oder verschiedenen C₁-C₄-Hydroxyalkylresten,
• eine Verbindung der folgenden Formel: 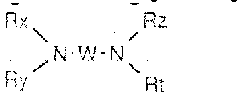 worin W für einen gegebenenfalls durch eine Hydroxylgruppe substituierten C₁-C₆-Alkylenrest steht; Rx, Ry, Rz und Rt gleich oder verschieden sind und für ein Wasserstoffatom oder einen C₁-C₆-Alkyl-, C₁-C₆-Hydroxyalkyl- oder C₁-C₆-Aminoalkylrest stehen;
handelt.

6. Verfahren nach einem der vorhergehenden Ansprüche, **dadurch gekennzeichnet, dass** es sich bei dem organischen Amin um ein Alkanolamin, vorzugsweise ausgewählt aus 2-Amino-2-methyl-1-propanol und Monoethanolamin oder Mischungen davon, handelt.

7. Verfahren nach einem der vorhergehenden Ansprüche, **dadurch gekennzeichnet, dass** das organische Amin aus den basischen Aminosäuren der folgenden Formel (I): 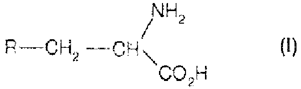 worin R für eine aus 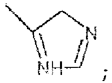 - (CH₂)₃NH₂; - (CH₂) ₂NH₂ ; - (CH₂) ₂NHCONH₂ ; 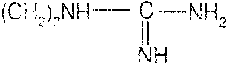 ausgewählte Gruppe steht, ausgewählt ist.

8. Verfahren nach einem der vorhergehenden Ansprüche, **dadurch gekennzeichnet, dass** das organische Amin aus Arginin, Histidin, Lysin oder Mischungen davon ausgewählt ist.

9. Verfahren nach einem der vorhergehenden Ansprüche, **dadurch gekennzeichnet, dass** der Gehalt an organischem Amin 0,1 bis 40 Gew.-% und vorzugsweise 0,5 bis 20 Gew.-%, bezogen auf das Gewicht der Zusammensetzung (B), ausmacht.

10. Verfahren nach einem der vorhergehenden Ansprüche, **dadurch gekennzeichnet, dass** die Gewichtsverhältnisse R1 der Mengen der Zusammensetzungen (A) + (C) / (B) und R2 der Mengen der Zusammensetzungen (A) / (C) von 0,1 bis 10 und vorzugsweise 0,3 bis 3 variieren.

## Claims

1. Process for dyeing human keratin fibres in the presence of an oxidizing agent, in which the following are applied to the said fibres:
(a) an anhydrous cosmetic composition (A) comprising one or more fatty substances and one or more surfactants; **characterized in that** the fatty substance(s) is (are) chosen from liquid petroleum jelly, polydecenes and liquid esters, or mixtures thereof, and **characterized in that** the fatty substance content is between 10% and 99% by weight relative to the weight of composition (A);
(b) a composition (B) comprising one or more oxidizing agents;
(c) a composition (C) comprising one or more oxidation dyes, one or more direct dyes or mixtures thereof and one or more organic amines whose pKb is less than 12 at 25°C.

2. Process according to any one of the preceding claims, **characterized in that** the surfactant present in composition (A) is a nonionic surfactant more particularly chosen from monooxyalkylenated or polyoxyalkylenated, monoglycerolated or polyglycerolated nonionic surfactants.

3. Process according to any one of the preceding claims, **characterized in that** the surfactant content represents from 0.1% to 50% by weight relative to the weight of the anhydrous composition (A).

4. Process according to any one of the preceding claims, **characterized in that** the organic amine comprises one or two primary, secondary or tertiary amine functions, and one or more linear or branched C₁-C₈ alkyl groups bearing one or more hydroxyl radicals.

5. Process according to the preceding claim, **characterized in that** the organic amine is:
• an alkanolamine chosen from mono-, di- and trialkanolamines, comprising from one to three identical or different C₁-C₄ hydroxyalkyl radicals,
• a compound having the following formula: 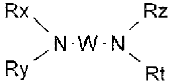 in which W is a C₁-C₆ alkylene residue optionally substituted with a hydroxyl group; Rx, Ry, Rz and Rt, which may be identical or different, represent a hydrogen atom or a C₁-C₆ alkyl, C₁-C₆ hydroxyalkyl or C₁-C₆ aminoalkyl radical.

6. Process according to any one of the preceding claims, **characterized in that** the organic amine is an alkanolamine, preferably chosen from 2-amino-2-methyl-1-propanol and monoethanolamine, or mixtures thereof.

7. Process according to any one of the preceding claims, **characterized in that** the organic amine is chosen from the basic amino acids of formula (I) below: 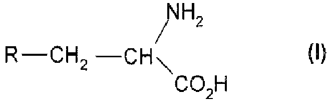 in which R denotes a group chosen from: 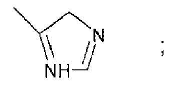 - (CH₂)₃NH₂ ; - (CH₂)₂NH₂ ; - (CH₂)₂NHCONH₂ ;

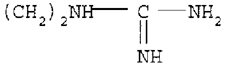

8. Process according to any one of the preceding claims, **characterized in that** the organic amine is chosen from arginine, histidine and lysine, or mixtures thereof.

9. Process according to any one of the preceding claims, **characterized in that** the organic amine content represents from 0.1% to 40% by weight and preferably from 0.5% to 20% by weight relative to the weight of composition (B).

10. Process according to any one of the preceding claims, **characterized in that** the weight ratios R1 of the amounts of compositions (A) + (C) / (B) and R2 of the amounts of compositions (A)/(C) vary from 0.1 to 10 and preferably from 0.3 to 3.
